# EUROPEAN PATENT APPLICATION

(11) **EP 0 867 453 A2**
(43) Date of publication of application: **30.09.1998**
(21) Application number: 98105659.1
(22) Date of filing: 27.03.1998
(51) Int. Cl.: C08B 37/08

(54) **A method of purifying sodium hyaluronate**

(30) Priority: 27.03.1997 JP 74859/97
(71) Applicant: KYOWA HAKKO KOGYO CO., LTD., Chiyoda-ku, Tokyo 100-8185 (JP)
(72) Inventor: Suga, Shigenori, Hofu-shi, Yamaguchi 747-0815 (JP); Muroi, Kenichi, Kitakyushu-shi, Fukuoka 800-0025 (JP); Kinoshita, Tadashi, Hofu-shi, Yamaguchi 747-0843 (JP); Goto, Joji, Zama-shi, Kanagawa 228-0026 (JP)
(74) Representative: VOSSIUS & PARTNER

(57) **Abstract**

Highly purified sodium hyaluronate having a low content of pyrogen is obtained by adding a salt and a water-soluble organic solvent to a solution containing sodium hyaluronate, or to a sodium hyaluronate solution previously filtered through a positively charged filter, precipitating and sedimenting the sodium hyaluronate and taking out a supernatant to remove pyrogen.

## Description

The present invention relates to a method of purifying sodium hyaluronate and to sodium hyaluronate obtained by said purification method.

Sodium hyaluronate being free of streptolysin and not showing hemolysis (Japanese Published Examined Patent Application No. 78116/91), sodium hyaluronate with low chemotaxis of human polymorphonuclear leukocytes and low ability to invade cells upon administration into the eyes of domestic rabbits (Japanese Published Examined Patent Application No. 8323/94), pyrogen-free, high-purity sodium hyaluronate (Japanese Published Unexamined Patent Application No. 501471/87), etc. are known.

Known industrial processes for production of sodium hyaluronate include a method of purifying an extract from cockscombs and a method of purifying said substance from a fermentation fluid obtained from a strain of the genus *Streptococcus.*

Known methods of removing high-molecular weight compounds such as pyrogen occurring as impurity in sodium hyaluronate include trichloroacetic acid treatment, chloroform-isoamyl alcohol treatment, enzyme treatment (Japanese Published Unexamined Patent Application No. 24194/85), ion-exchange resin treatment (Japanese Published Unexamined Patent Application No. 12293/88), adsorption resin treatment (Japanese Published Unexamined Patent Application No. 103203/90), methods of selectively precipitating sodium hyaluronate with cetyl pyridium chloride (Japanese Published Unexamined Patent Application No. 133894/85) or with a large amount of water-soluble organic solvent, etc. (Japanese Published Unexamined Patent Application No. 268765/90) and a method of removing pyrogen by passing a solution with viscosity of 20,000 to 60,000 centistoke through a filter, positively charged in a solution at pH 6 to 10 (Japanese Unexamined Patent Application No. 199656/94).

Sodium hyaluronate having physical properties such as lack of hemolysis, absence of chemotaxis and negativity in a pyrogen test has been provided. However, complicated operations have been required for removal of pyrogen from sodium hyaluronate.

A purification method of easily removing pyrogen from sodium hyaluronate has been desired.

The object of the present invention is to provide a purification method for easily obtaining high-purity sodium hyaluronate having a low content of pyrogen, as well as sodium hyaluronate obtained by said purification method.

According to the present invention, there can be provided a method of purifying sodium hyaluronate which comprises adding salt and a water-soluble organic solvent to a solution containing sodium hyaluronate, precipitating and sedimenting the sodium hyaluronate, and taking out the supernatant to remove pyrogen, as well as sodium hyaluronate obtained by said purification method.

In addition, there can be provided a method of purifying sodium hyaluronate which comprises filtering a solution containing sodium hyaluronate through a positively charged filter and then removing pyrogen in the above-described method, as well as sodium hyaluronate obtained by said purification method.

Further, there can be provided a method of purifying sodium hyaluronate which comprises washing sodium hyaluronate obtained by the above-described method with at least 50 % (v/v) water-soluble organic solvent, pulverizing sodium hyaluronate, and drying sodium hyaluronate under reduced pressure to further reduce pyrogen, as well as sodium hyaluronate obtained by said purification method.

Sodium hyaluronate which can be used in the present invention for being purified may be any sodium hyaluronate regardless of whether it was derived from microorganism, animal or any other source and regardless what molecular weight it has.

The sodium hyaluronate includes, for example, one isolated from vitreous body, umbilical cord, synovia, cockscombs etc. and one produced by microorganisms such as *Streptococcus pyogenes, Streptococcus equi, Streptococcus equismilis, Streptococcus dysgalactiae* and *Streptococcus zooepidemicus.*

The sodium hyaluronate solution is preferably an aqueous solution containing sodium hyaluronate at a concentration of 0.001 to 1 % (w/v), and water for dissolving sodium hyaluronate is preferably water with 0.01 EU/ml or less pyrogen.

To remove microorganisms or insolubles, the sodium hyaluronate solution may be subjected to filtration treatment with a 0.2 to 5.0 µm filter.

For the purpose of removing pyrogen from the sodium hyaluronate solution, a filter positively charged at a specific pH region may be used for filtration treatment.

The filter may be any filter so long as it is positively charged in an aqueous solution at pH 3 to 10. A preferable example is a membrane filter.

The filter is preferably a filter with a membrane pore size of 0.20 to 5.0 µm composed of a copolymer having polyamide resin such as nylon-66 as one component.

Examples of such filters include Posidine™ filter, Profile 11 Plus™ which are produced by Nippon Paul K.K., and the like.

Where the filtration treatment is carried out using said filter, the operation of adding salts or raising the temperature of the hyaluronate solution may be carried out.

The salt includes those composed of cationic components consisting of alkali metal ions such as lithium, sodium and potassium, alkaline earth metal ions such as magnesium and calcium, and aluminum ion, etc; and anionic components consisting of halogen ions such as chlorine and bromine, acid roots of inorganic acids such as sulfuric acid and nitric acid, and organic acids such as formic acid and acetic acid as acid components, and preferable examples are sodium salts. These salts can be used singly or in combination thereof.

Specific examples of salts include sodium chloride, sodium sulfate, sodium acetate etc.

The salt is added to the hyaluronate solution preferably in an amount of 0.1 to 30 % (w/v).

The temperature of the hyaluronate solution can be raised up to 80 °C.

A salt and water-soluble organic solvent are added to the sodium hyaluronate solution which is subjected to the above-described filter treatment or to the sodium hyaluronate solution which is not subjected to said treatment.

As the salt, the above-described salt can be used singly or in combination thereof.

The salt is added to the hyaluronate solution preferably in an amount of 1 to 30 % (w/v).

The water-soluble organic solvent is added preferably at a final concentration of 30 to 90 % (v/v) at which sodium hyaluronate can be precipitated.

The water-soluble organic solvent used may be any water-soluble organic solvent and includes, for example, alchols such as methanol, ethanol, n-propanol and isopropanol, ketones such as acetone, ethers such as dimethoxyethane, tetrahydrofuran and dioxane, acetonitrile, etc. These water-soluble organic solvents can be used singly or in a mixture thereof. Further, the water-soluble organic solvent used is preferably one having a low content of pyrogen.

To effectively remove pyrogen from sodium hyaluronate precipitated and sedimented by adding the water-soluble organic solvent to the sodium hyaluronate solution, it is preferable to efficiently sediment sodium hyaluronate, usually by leaving the solution for at least 2 hours after addition of the water-soluble organic solvent so that sodium hyaluronate is sedimented. The sodium hyaluronate sedimented in this step is in the form of semi-transparent gel of low specific volume.

Pyrogen can be removed by taking out a supernatant from the solution from which said sodium hyaluronate was sedimented.

From the sodium hyaluronate remaining after taking out the supernatant, pyrogen can be further removed in the following manner.

The sodium hyaluronate in the form of gel after taking out the supernatant is washed by adding 50 to 100 % (v/v) water-soluble organic solvent to the sodium hyaluronate. By this operation, the sodium hyaluronate turns from gel to powder. If necessary, said powdery sodium hyaluronate may be washed by further adding 50 to 100 % (v/v) water-soluble organic solvent followed by vacuum drying whereby sodium hyaluronate having a pyrogen content of 0.0015 to 0.003 EU/mg or less can be obtained.

For measurement of its pyrogen, the sodium hyaluronate is dissolved in water having a pyrogen content of 0.006 EU/ml or less and then subjected to colorimetric analysis using Toxi Color System (Seikagaku Kogyo K.K.) in accordance with the manufacture's instructions.

According to the present invention, there can be provided a purification method for easily obtaining high-purity sodium hyaluronate having a low content of pyrogen, as well as sodium hyaluronate obtained by said purification method.

### Examples

Hereinafter, the examples of the present invention are shown.

### Example 1

First, 25g of sodium hyaluronate having a pyrogen content of 0.02 EU/mg was dissolved in 10 L water having a pyrogen content of 0.01 EU/ml or less and then the mixture was filtered through a 0.45 µm microfilter to give a filtrate.

Sodium chloride was added up to 58.5 g/l to the filtrate and then ethanol was added thereto at 50 or 44 % (v/v).

The solution was allow to stand for about 5 hours to precipitate and sediment sodium hyaluronate, and the supernatant was taken out from the solution so that sodium hyaluronate precipitates were obtained.

The precipitates were washed with 80 % aqueous ethanol and then with 100 % ethanol.

The precipitates thus washed were dried under reduced pressure to give 20 g purified sodium hyaluronate.

The content of pyrogen in the sodium hyaluronate thus obtained is shown in Table 1.

**Table 1**

| Ethanol concentration at the time of precipitation (v/v%) | Pyrogen content (EU/mg) |
|---|---|
| 50 | 0.005 |
| 44 | 0.003 or less |

### Example 2

First, 25 g of sodium hyaluronate having a pyrogen content of 0.02 EU/mg was dissolved in 10 L water having a pyrogen content of 0.01 EU/ml or less and then the mixture was filtered through a 0.45 µm microfilter to give a filtrate and then sodium chloride was added up to 58.5 g/l to the filtrate and acetone was added thereto at 50, 44, or 41 % (v/v).

The solution was allow to stand for about 5 hours to precipitate and sediment sodium hyaluronate, and the supernatant was removed from the solution so that sodium hyaluronate precipitates were obtained.

The precipitates were washed with 80 % acetone and then with 100 % acetone.

The resulting precipitates were dried under reduced pressure to give 20 g of purified sodium hyaluronate.

The content of pyrogen in the sodium hyaluronate thus obtained is shown in Table 2.

**Table 2**

| Acetone concentration at the time of precipitation (v/v%) | Pyrogen content (EU/mg) |
|---|---|
| 50 | 0.010 |
| 44 | 0.003 |
| 41 | 0.003 or less |

### Example 3

*Streptococcus zooepidemicus* NCTC7023 [J. Gen. Microbiol., 15, 485-491 (1956)] was cultivated in a brain heart infusion agar medium (produced by Nissui Seiyaku K.K.) at 37 °C for 16 hours and then inoculated into 300 ml of a medium, pH 7.0 consisting of 1 % glucose, 1.5 % peptone, 0.5 % yeast extract, 1 % corn steep liquor, 0.3 % sodium glutamate, 0.2 % dipotassium phosphate, 0.05 % magnesium sulfate, 0.1 % sodium thiosulfate, and 2 % calcium carbonate and cultivated at 37 °C for 16 hours under shaking.

Then, 150 ml of the resulting culture was inoculated into a 5 L-jar fermentor containing 3 L of a fermentation medium, pH 7.2 consisting of 2.5 % glucose, 1.5 % peptone, 0.5 % yeast extract, 0.5 % corn steep liquor, 0.2 % dipotassium phosphate, 0.005 % magnesium sulfate and 0.1 % sodium thiosulfate, and cultivated at 37 °C under aeration at 0.3 vvm at pH 7.0 for 26 hours to give a culture containing hyaluronate.

Then, 3 L of the culture containing hyaluronate was diluted with ion-exchanged water until its volume reached 20 L, then 200 g of carbon was added thereto, and the mixture was stirred at room temperature for 1 hour and filtrated through a nuche to give 20 L of carbon-treated solution.

The solution thus treated with carbon was passed successively through 2.5 L of micro-reticular type anion exchange resin Diya Ion HPA-75 (Mitsubishi Chemical Corporation) and then through 1.25 L of gel type strongly acidic cation exchange resin SK1B (Mitsubishi Chemical Corporation) at a flow rate of 5 L/h and 20 L of a solution was obtained after chromatography.

The resulting solution was adjusted to pH 7.0 with sodium hydroxide, and 200 g of carbon was added thereto, and the mixture was stirred at room temperature for 1 hour.

After stirring, it was filtered through a nuche, and the resulting filtrate was filtered through a 0.45 µm microfilter to give 20 L of a filtrate.

The content of pyrogen in this filtrate was 0.04 EU per mg of sodium hyaluronate.

Then, 1.2kg of sodium chloride was added to the filtrate, followed by adding 14 L of acetone.

The solution was allowed to stand for about 5 hours whereby sodium hyaluronate was precipitated and sedimented, and the supernatant was removed from the solution whereby sodium hyaluronate precipitates were obtained.

The precipitates were washed with 80 % acetone and then with 100 % acetone.

The precipitates thus washed were dried under reduced pressure to give 11.5 g of purified sodium hyaluronate.

The content of pyrogen in the sodium hyaluronate thus obtained is shown in Table 3.

**Table 3**

| Acetone concentration at the time of precipitation (v/v%) | Pyrogen content (EU/mg) |
|---|---|
| 41 | 0.003 or less |

### Example 4

First, 25 g of sodium hyaluronate having a pyrogen content of 0.02 EU/ml, derived from cockscombs, was dissolved in 10 L of water having a pyrogen content of 0.01 EU/ml or less and then the mixture was filtered through a 0.45 µm microfilter to give a filtrate.

Sodium chloride was added up to 58.5 g/l to the filtrate, and then acetone was added thereto at 41 % (v/v).

The solution was allowed to stand for about 5 hours to precipitate and sediment sodium hyaluronate, and the supernatant was removed from the solution so that sodium hyaluronate precipitates were obtained.

The precipitates were washed with 80 % acetone and then with 100 % acetone.

The precipitates thus washed were dried under reduced pressure to give 20 g of purified sodium hyaluronate.

The content of pyrogen in the sodium hyaluronate thus obtained is shown in Table 4.

**Table 4**

| Acetone concentration at the time of precipitation (v/v%) | Pyrogen content (EU/mg) |
|---|---|
| 41 | 0.003 or less |

### Example 5

First, 25 g of sodium hyaluronate having molecular weight of 2,300,000 and containing 0.02 EU/ml pyrogen was dissolved in 10 L of water having a pyrogen content of 0.01 EU/ml or less and then sodium chloride was added up to 10 g/l to the solution.

The hyaluronate solution was passed through a microfilter positively charged at pH 3 to 10 [absolute filtration accuracy 0.45 µm, Posidine Filter™, a product of Nippon Paul K.K.] at a filtration temperature of 25 °C to give a filtrate.

Sodium chloride was added up to 58.5 g/l to the filtrate, and then ethanol was added thereto at 44 % (v/v).

The solution was allowed to stand for about 5 hours to precipitate and sediment sodium hyaluronate, and the supernatant was removed from the solution so that sodium hyaluronate precipitates were obtained.

The precipitates were washed with 80 % aqueous ethanol and then with 100 % ethanol.

The precipitates thus washed were dried under reduced pressure to give 20 g of purified sodium hyaluronate.

The content of pyrogen in the sodium hyaluronate thus obtained is shown in Table 5.

**Table 5**

| Pyrogen Content (EU/mg) | | |
|---|---|---|
| Solution | Membrane filtrate | Sodium hyaluronate |
| 0.020 | 0.005 | 0.0015 or less |

### Example 6

First, 50 g of sodium hyaluronate having molecular weight of 600,000 and containing 0.02 EU/ml pyrogen was dissolved in 10 L of water having a pyrogen content of 0.01 EU/ml or less and then sodium chloride was added up to 10 g/l to the solution.

The hyaluronate solution was passed through a membrane filter positively charged at pH 3 to 10 [absolute filtration accuracy 0.45 µm, Posidine Filter™, a product of Nippon Paul K.K.] at a filtration temperature of 25 °C to give a filtrate.

Sodium chloride was added up to 58.5 g/l to the filtrate, and then ethanol was added thereto at 44 % (v/v).

The solution was allowed to stand for about 5 hours to precipitate and sediment sodium hyaluronate, and the supernatant was removed from the solution so that sodium hyaluronate precipitates were obtained.

The precipitates were washed with 80 % aqueous ethanol and then with 100 % ethanol.

The precipitates thus washed were dried under reduced pressure to give 40 g of purified sodium hyaluronate.

The content of pyrogen in the sodium hyaluronate thus obtained is shown in Table 6.

**Table 6**

| Pyrogen Content (EU/mg) | | |
|---|---|---|
| Solution | Membrane filtrate | Sodium hyaluronate |
| 0.020 | 0.005 | 0.0015 or less |

### Example 7

*Streptococcus zooepidemicus* NCTC7023 [J. Gen. Microbiol., 15, 485-491 (1956)] was cultivated in a brain heart infusion agar medium (produced by Nissui Seiyaku K.K.) at 37 °C for 16 hours and then inoculated into 300 ml of a medium, pH 7.0 consisting of 1 % glucose, 1.5 % peptone, 0.5 % yeast extract, 1 % corn steep liquor, 0.3 % sodium glutamate, 0.2 % dipotassium phosphate, 0.05 % magnesium sulfate, 0.1 % sodium thiosulfate, and 2 % calcium carbonate and cultivated at 37 °C for 16 hours under shaking.

Then, 150 ml of the resulting culture was inoculated into a 5 L jar fermentor containing 3 L of fermentation medium, pH 7.2 consisting of 2.5 % glucose, 1.5 % peptone, 0.5 % yeast extract, 0.5 % corn steep liquor, 0.2 % dipotassium phosphate, 0.005 % magnesium sulfate and 0.1 % sodium thiosulfate, and cultivated at 37 °C under aeration at 0.3 vvm at pH 7.0 for 26 hours to give a culture containing hyaluronate.

After the cultivation, 3 L of the culture containing hyaluronate was diluted with ion-exchanged water until its volume reached 20 L, and 200 g of carbon was added thereto, and the mixture was stirred at room temperature for 1 hour and then filtrated through a nuche to give 20 L of a carbon-treated solution.

The solution thus treated with carbon was passed successively through 2.5 L micro-reticular type anion exchange resin Diya Ion HPA-75 (Mitsubishi Chemical Corporation) and then through 1.25 L gel type strongly acidic cation exchange resin SK1B (Mitsubishi Chemical Corporation) at a flow rate of 5L/h, and 20 L of a solution was obtained after chromatography.

The resulting solution was adjusted to pH 7.0 with sodium hydroxide, and 200 g of carbon was added thereto, and the mixture was stirred at room temperature for 1 hour and filtered through a nuche to give a filtrate.

Sodium chloride was added up to 10 g/l to the filtrate.

The hyaluronate solution was filtered through a membrane filter positively charged at pH 3 to 10 [absolute filtration accuracy 0.45 µm, Posidine Filter™, a product of Nippon Paul K.K.] at a filtration temperature of 25 °C to give 18 L of filtrate.

The pyrogen content in the filtrate was 0.01 EU per mg of sodium hyaluronate.

Then, 1.1 kg sodium chloride was added to the filtrate, and then 14 L of ethanol was added thereto.

The solution was allowed to stand for about 5 hours to precipitate and sediment sodium hyaluronate, and the supernatant was removed from the solution so that sodium hyaluronate precipitates were obtained.

The precipitates were washed with 80 % ethanol and then with 100 % ethanol.

The precipitates thus washed were dried under reduced pressure to give 10.0 g of purified sodium hyaluronate. The molecular weight of the sodium hyaluronate was 2,200,000 when analyzed by the intrinsic viscosity method.

The content of pyrogen in the sodium hyaluronate thus obtained is shown in Table 7.

**Table 7**

| Pyrogen Content (EU/mg) |
|---|
| 0.0015 or less |

### Example 8

First, 50 g of sodium hyaluronate having molecular weight of 1,000,000, derived from cockscombs and containing 0.02 EU/ml pyrogen was dissolved in 10 L of water having a pyrogen content of 0.01 EU/ml or less and then sodium chloride was added up to 10 g/l to the solution.

The hyaluronate solution was filtered through a membrane filter positively charged at pH 3 to 10 [absolute filtration accuracy 0.45 µm, Posidine Filter™, a product of Nippon Paul K.K.] at a filtration temperature of 25 °C to give a filtrate.

Sodium chloride was added up to 58.5 g/l to the filtrate, and then acetone was added thereto at 41 % (v/v).

The solution was allowed to stand for about 5 hours to precipitate and sediment sodium hyaluronate, and the supernatant was removed from the solution so that sodium hyaluronate precipitates were obtained.

The precipitates were washed with 80 % acetone and then with 100 % acetone.

The precipitates thus washed were dried under reduced pressure to give 40 g of purified sodium hyaluronate.

The content of pyrogen in the sodium hyaluronate thus obtained is shown in Table 8.

**Table 8**

| Solution | Membrane filtrate | Sodium hyaluronate |
|---|---|---|
| 0.020 | 0.005 | 0.0015 or less |

### Example 9

First, 10g of sodium hyaluronate having molecular weight of 2,300,000 and containing 0.1 EU/ml pyrogen was dissolved in 10 L of water having a pyrogen content of 0.01 EU/ml or less and then sodium chloride was added up to 10 g/l to the solution.

The hyaluronate solution was filtered through a microfilter positively charged at pH 3 to 10 [absolute filtration accuracy 0.2 µm, Posidine Filter™, a product of Nippon Paul K.K.] at a filtration temperature of 25 °C to give a filtrate.

Sodium chloride was added up to 58.5 g/l to the filtrate, and then ethanol was added thereto at 44 % (v/v).

The solution was allowed to stand for about 5 hours to precipitate and sediment sodium hyaluronate, and the supernatant was removed from the solution so that sodium hyaluronate precipitates were obtained.

The precipitates were washed with 80 % aqueous ethanol and then with 100 % ethanol.

The precipitates thus washed were dried under reduced pressure to give 2 g of purified sodium hyaluronate.

The content of pyrogen in the sodium hyaluronate thus obtained is shown in Table 9.

**Table 9**

| Solution | Sodium hyaluronate |
|---|---|
| 0.1 | 0.0015 or less |

The present invention can provide a purification method for easily obtaining high-purity sodium hyaluronate having a low content of pyrogen, as well as sodium hyaluronate obtained by the purification method.

## Claims

1. A method of purifying sodium hyaluronate, which comprises adding a salt and a water-soluble organic solvent to a solution containing sodium hyaluronate, precipitating and sedimenting the sodium hyaluronate and taking out a supernatant to remove pyrogen.

2. The purification method according to claim 1, wherein the content of sodium hyaluronate in the solution is in the range of 0.001 to 1 % (W/V).

3. The purification method according to claim 1, wherein the sodium hyaluronate is sodium hyaluronate derived from a cockscomb or microorganism.

4. The purification method according to claim 3, wherein the microorganism is a microorganism belonging to the genus *Streptococcus.*

5. The purification method according to claim 4, wherein the microorganism belonging to the genus *Streptococcus* is *Streptococcus zooepidemicus* NCTC7023.

6. The purification method according to claim 1, wherein the salt is added in an amount of 1 to 30 % (W/V).

7. The purification method according to claim 1 or 6, wherein the salt is a sodium salt.

8. The purification method according to claim 7, wherein the sodium salt is a salt selected from sodium chloride, sodium sulfate and sodium acetate.

9. The purification method according to claim 1, wherein the water-soluble organic solvent is added in an amount of 30 to 45 % (V/V).

10. The purification method according to claim 1, wherein the water-soluble organic solvent is methanol, ethanol or acetone.

11. The purification method according to claim 1 or 2, wherein the solution containing sodium hyaluronate is a sodium hyaluronate solution having been filtered through a microfilter positively charged in a solution at pH 3 to 10.

12. The purification method according to claim 11, wherein the pore size of the positively charged microfilter is in the range of 0.20 to 5.0 µm.

13. The purification method according to claim 11, wherein the sodium hyaluronate solution contains a salt.

14. The purification method according to claim 13, wherein the salt is added in an amount of 0.1 to 30 % (W/V).

15. The purification method according to claim 13 or 14, wherein the salt is a sodium salt.

16. The purification method according to claim 15, wherein the sodium salt is a salt selected from sodium chloride, sodium sulfate and sodium acetate.

17. A method of purifying sodium hyaluronate, which comprises adding a 50 to 100 % (V/V) water-soluble organic solvent to sodium hyaluronate obtained in the purification method described in any one of claims 1 to 16, washing sodium hyaluronate with the solvent and drying sodium hyaluronate under reduced pressure.

18. Sodium hyaluronate obtainable by the purification method described in any one of claims 1 to 17.

19. Sodium hyaluronate of which the pyrogen content is 0.003 endotoxin units (EU)/mg.
